# DEMANDE DE BREVET EUROPEEN

(11) **EP 1 849 457 A1**
(43) Date de publication de la demande: **31.10.2007**
(21) Numéro de dépôt: 07106949.6
(22) Date de dépôt: 25.04.2007
(51) Int. Cl.: A61K 8/73, A61K 8/60, A61Q 5/02

(54) **Composition cosmétique comprenant un ester de sorbitol oxyéthyléné et une cyclodextrine, procédés et utilisations**

(30) Priorité: 27.04.2006 FR 0603808
(71) Demandeur: L'Oréal, 75008 Paris (FR)
(72) Inventeur: Müller, Rainer, 76344 Leopoldshafen (DE)
(74) Mandataire: Bulle, Françoise

(57) **Abrégé**

La présente demande concerne une composition cosmétique comprenant, dans un milieu aqueux physiologiquement acceptable, au moins un ester d'acide gras en C₈ à C₁₄ et de sorbitan oxyéthyléné comprenant de 2 à 10 unités d'oxyde d'éthylène et au moins une cyclodextrine.

La présente demande concerne également un procédé de traitement cosmétique, en particulier de lavage et de soin des fibres kératiniques telles que les cheveux, mettant en oeuvre ladite composition, ainsi que les utilisations de ces compositions cosmétiques en particulier pour le traitement tel que le lavage des fibres kératiniques en particulier des cheveux.

## Description

La présente demande se rapporte au domaine des compositions cosmétiques notamment des compositions cosmétiques lavantes telles que les shampooings comprenant des agent nacrants.

De nombreuses compositions de lavage des matières kératiniques ont été décrites dans l'art antérieur.

Outre leurs propriétés lavantes et leurs qualités cosmétiques, les utilisateurs sont sensibles au fait que les compositions lavantes, en particulier les shampooings, n'agressent pas la peau ni les yeux, cette qualité est en particulier appréciée dans les shampooings pour enfants.

Par ailleurs, les produits cosmétiques ayant un aspect ou un effet irisé, moiré ou métallisé, sont largement appréciés par les consommateurs pour leur aspect esthétique car ils donnent une apparence de richesse au produit. Les agents qui apportent cet effet sont des agents de nacrage ou agent nacrants comprenant généralement des cristaux qui restent dispersés dans les compositions et qui réfléchissent la lumière.

Les dérivés d'esters à longue chaîne sont largement utilisés pour nacrer les compositions notamment cosmétiques. Cependant, ces dérivés peuvent présenter des problèmes de cristallisation qui entraînent une évolution de la viscosité des compositions au cours du temps.

On connaît également les dérivés d'éthers ou de thioéthers à longue chaîne tels que ceux décrits dans les demandes EP 457 688 et WO 98/03155. Cependant, ces derniers agents opacifient les compositions sans apporter de nacrage aux compositions ou pas suffisamment.

Il a été aussi constaté que ces agents nacrants, du fait de leur faible densité, présentaient souvent l'inconvénient de remonter à la surface du shampooing et d'y former une couche inesthétique pour le consommateur.

De plus ces composés à chaînes grasses présentent, dans certains cas, l'inconvénient d'apporter un toucher chargé aux cheveux, un manque de légèreté et de volume de la chevelure.

Tous ces agents nacrants sont des composés insolubles dans l'eau et ont un point de fusion supérieur à 50°C. Pour fabriquer des compositions nacrées, il faut donc chauffer les compositions au-dessus du point de fusion du composé nacrant puis refroidir et ensuite ajouter les autres composés de la composition. Afin de diminuer la consommation d'énergie et de réduire la durée de fabrication, il est souhaitable de préparer les compositions à froid.

La demande WO 03/088934 décrit l'utilisation de cyclodextrine comme agent nacrant qui peut être utilisé à froid. Cependant les compositions contenant une cyclodextrine ne sont pas encore suffisamment stables.

Le problème posé dans la présente demande est l'obtention de compositions cosmétiques nacrées, notamment lavantes telles que des shampooings, qui soient stables dans le temps et qui ne soient pas agressives pour la peau et les yeux.

Par « stables » au sens de la présente demande, on entend que l'aspect visuel et le toucher de ces compositions n'évoluent sensiblement pas au cours du temps dans des conditions normales de stockage, par exemple pendant les 12, de préférence 24 et de manière encore préférée 30 mois qui suivent leur fabrication. En particulier, on n'observe pas de déphasage des compositions selon la présente demande, les agents nacrants contenus dans ces compositions ne remontent pas à la surface.

Par « non agressive » au sens de la présente demande, on entend que la composition ne provoque pas de picotements dans l'oeil lorsqu'elle atteint la sphère oculaire et plus généralement, ne provoque pas de réactions telles que rougeurs, démangeaisons, picotements notamment chez les utilisateurs présentant une peau sensible.

Par « matières kératiniques », on comprend les cheveux, les cils, les sourcils, la peau, les ongles, les muqueuses ou le cuir chevelu et plus particulièrement les cheveux et par« fibres kératiniques », on comprend les cheveux, les cils, les sourcils.

Par le terme "agent de nacrage" ou "agent nacrant", on entend un agent produisant un aspect ou un effet nacré, irisé, moiré ou métallisé.

Par « composition lavante » au sens de la présente demande, on entend une composition comprenant au moins % en poids d'au moins un tensioactif, de préférence entre 4 % et 50 % en poids d'au moins un tensio-actif par rapport au poids total de la composition.

La demanderesse a découvert qu'il était possible de formuler des compositions cosmétiques stables et non agressives pour le traitement des matières kératiniques, en particulier des shampooings, présentant un aspect nacré tout en ayant les propriétés lavantes, esthétiques et cosmétiques recherchées, en utilisant au moins un ester d'acide gras en C₈ à C₁₄ et de sorbitan oxyéthyléné comprenant de 2 à 10 unités d'oxyde d'éthylène (ou OE) et au moins une cyclodextrine ou un de ses dérivés.

La présente demande concerne donc une composition cosmétique, en particulier une composition lavante telle qu'un shampooing, comprenant dans un milieu aqueux physiologiquement acceptable au moins un ester d'acide gras en C₈ à C₁₄ et de sorbitan oxyéthyléné comprenant de 2 à 10 unités d'oxyde d'éthylène et au moins une cyclodextrine ou un de ses dérivés. La présente demande concerne également un procédé de traitement cosmétique, en particulier de lavage des fibres kératiniques telles que les cheveux, mettant en oeuvre ladite composition, ainsi que les utilisations de ces compositions cosmétiques en particulier pour le traitement tel que le lavage des fibres kératiniques en particulier des cheveux.

La présente demande vise encore les utilisations de la composition selon la présente demande notamment comme composition de traitement ou de soin cosmétique des matières kératiniques et/ou comme shampooing ou composition à appliquer avant ou après un shampooing.

Les compositions selon la présente demande présentent une très bonne homogénéité et une bonne stabilité du nacrage, pour l'application sur les matières kératiniques. Il n'y a pas de déphasage (séparation de phase).

En particulier, il ne se produit aucun relargage ou épaississement incontrôlé de la composition au cours du temps. Les compositions présentent enfin une texture non filante et fondante. La mousse se rince facilement.

D'autres caractéristiques, aspects, objets et avantages de la présente invention apparaîtront encore plus clairement à la lecture de la description et des exemples qui suivent.

La composition objet de la présente invention comprend un agent tensio-actif non ionique particulier, constitué d'au moins un ester d'acide gras en C₈ à C₁₄ et de sorbitan oxyéthyléné comprenant de 2 à 10 unités d'oxyde d'éthylène.

De manière préférée, la composition selon l'invention comprend au moins un ester d'acide gras en C₁₂ et de sorbitan oxyéthyléné comprenant de 2 à 10 unités d'oxyde d'éthylène, et de préférence 4 unités d'oxyde d'éthylène.

De manière encore plus préférée, la composition selon l'invention comprend du mono-laurate de sorbitan oxyéthyléné à 4 OE. Ce composé est également connu sous le nom de polysorbate 21. Il est, entre autres, commercialisé sous la dénomination TWEEN 21 par la société UNIQEMA.

La composition selon l'invention comprend préférentiellement au moins 0,5 % en poids d'ester d'acide gras en C₈ à C₁₄ et de sorbitan oxyéthyléné comprenant de 2 à 10 unités d'oxyde d'éthylène, par rapport au poids total de la composition. De préférence, elle comprend de 0,5 à 10 % en poids d'ester d'acide gras en C₈ à C₁₄ et de sorbitan oxyéthyléné comprenant de 2 à 10 unités d'oxyde d'éthylène, plus préférentiellement de 2 à 9 % en poids, encore plus préférentiellement de 4 à 8 % en poids, par rapport au poids total de la composition.

L'ester d'acide gras en C₈ à C₁₄ et de sorbitan oxyéthyléné comprenant de 2 à 10 unités d'oxyde d'éthylène peut être utilisé seul, ou en mélange avec un ou plusieurs autres dérivés oxyéthylénés du sorbitan. Par exemple, il est avantageux d'employer également dans les compositions selon l'invention au moins un monoester d'acide gras en C₈ à C₂₄ et de sorbitan oxyéthyléné comprenant de 15 à 50 unités d'oxyde d'éthylène.

Ces compositions présentent l'avantage supplémentaire d'avoir un aspect nacré amélioré, visible à l'oeil nu, et un nacrage persistant dans le temps, tout en ayant les propriétés esthétiques recherchées. Les compositions sont notamment moins jaunies.

De préférence, ledit monoester est un monoester d'acide gras en C₈ à C₂₄ et de sorbitan oxyéthyléné comprenant 20 unités d'oxyde d'éthylène.

De manière encore plus préférée, ledit monoester est le mono-laurate de sorbitan oxyéthyléné à 20 OE.

La composition selon l'invention comprend préférentiellement de 0,1 à 10 % en poids de monoester d'acide gras en C₈ à C₂₄ et de sorbitan oxyéthyléné comprenant de 15 à 50 unités d'oxyde d'éthylène, plus préférentiellement de 0,5 à 5 % en poids, par rapport au poids total de la composition.

Les cyclodextrines utilisables à titre d'agent nacrant dans les compositions selon la présente demande sont notamment des oligosaccharides de formule : 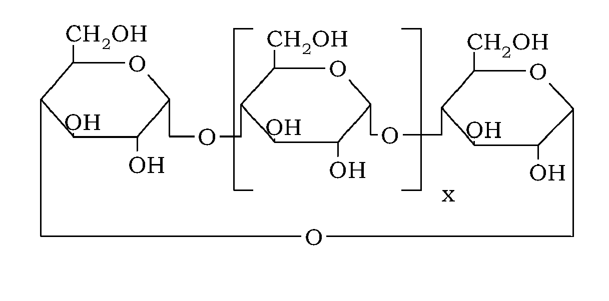 dans laquelle x peut être un nombre égal à 4 (ce qui correspond à l'α-cyclodextrine), à 5 (β-cyclodextrine) ou à 6 (γ-cyclodextrine).

De préférence la cyclodextrine est choisie parmi la β-cyclodextrine et la γ-cyclodextrine, de manière préférée il s'agit de la β-cyclodextrine.

On peut notamment utiliser une beta-cyclodextrine vendu par la société WACKER sous la dénomination CAVAMAX W7 PHARMA et une gamma cyclodextrine vendu par la société WACKER sous la dénomination CAVAMAX W8.

Les dérivés de cyclodextrines sont par exemple les méthyl cyclodextrines tels que la méthyl-beta-cyclodextrine commercialisée par la société WACKER sous la dénomination CAVASOL W7).

Selon l'invention, la ou les cyclodextrines ou un leurs dérivés représentent de 1 à 15 % en poids, de préférence de 1 % à 10 % en poids, et encore plus préférentiellement de 1,5 % à 5 % par rapport au poids total de la composition.

La composition selon la présente demande peut contenir un ou plusieurs tensioactifs additionnels. La cyclodextrine ou l'un de ses dérivés et l'ensemble des tensioactifs (y compris le ou les esters de sorbitol oxyéthylénés) sont présents en une concentration efficace pour nacrer la composition et/ou pour former un complexe insoluble dans la composition entre la cyclodextrine et le ou les tensioactifs.

De préférence, la cyclodextrine est introduite dans la composition sous forme non complexée ou éventuellement complexée avec le tensioactif ou les tensioactifs, c'est-à-dire que lorsque qu'un complexe est formé, il n'a pas été formé avec un autre composé que les tensioactifs.

Dans la composition, la cyclodextrine est de préférence complexée avec le ou les tensioactifs.

Le rapport pondéral ensemble des tensioactifs(y compris le ou les esters de sorbitol oxyéthylénés)/cyclodextrine peut varier de 0,01 à 300, de préférence de 0,1 à 100 et plus particulièrement de 0,3 à 25.

A titre de tensio-actifs additionnels utilisables dans les compositions de l'invention, on peut citer les tensioactifs classiquement utilisés dans ce domaine tels que les tensioactifs anioniques, non-ioniques additionnels autres que les dérivés oxyéthylénés du sorbitan, amphotères ou zwitterioniques, ou cationiques.

Les tensioactifs anioniques pouvant être utilisés dans les compositions selon l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆-C₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆-C₂₄)éther-carboxyliques polyoxyalkylénés, les acides (alkyl en C₆-C₂₄)(aryl en C₆-C₂₄)éther-carboxyliques polyoxyalkylénés, les acides (alkyl en C₆-C₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,5 à 50 % en poids, mieux encore de 4 à 20 % en poids par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du saccharose, les esters d'acides gras du polyéthylèneglycol, les (alkyl en C₆-C₂₄)polyglycosides, les dérivés de N-(alkyl en C₆-C₂₄)glucamine, les oxydes d'amines tels que les oxydes d'(alkyl en C₁₀-C₁₄)amines ou les oxydes de N-(acyl en C₁₀-C₁₄)-aminopropylmorpholine.

Lorsqu'ils sont présents, la quantité du ou des tensioactifs non ioniques additionnels est de préférence comprise dans l'intervalle allant de 0,01 à 10 % en poids, mieux encore de 0,05 à 5 % en poids par rapport au poids total de la composition.

Les compositions de l'invention peuvent également comprendre un ou plusieurs tensioactifs amphotères ou zwittérioniques.

Les agents tensioactifs amphotères ou zwittérioniques utilisables dans les compositions selon l'invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl(C₈-C₂₀)bétaïnes, les sulfobétaïnes, les (alkyl en C₈-C₂₀)amido(alkyl en C₆-C₈)bétaïnes ou les (alkyl en C₈-C₂₀)amido(alkyl en C₆-C₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (I) et (II) :

Rₐ-CONHCH₂CH₂-N(R_{b})(R_{c})(CH₂COO⁻) (I)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(B') (II)
dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈-C₂₀)bétaïnes, les (alkyl en C₈-C₂₀)amido(alkyl en C₆-C₈)bétaïnes et leurs mélanges.

Lorsqu'ils sont présents, la quantité du ou des tensioactifs amphotères ou zwittérioniques est de préférence comprise dans l'intervalle allant de 0,1 à 10 % en poids, mieux encore de 0,5 à 8 % en poids par rapport au poids total de la composition.

La composition selon l'invention présente de préférence une teneur totale en tensioactifs anioniques, non-ioniques, amphotères et zwittérioniques comprise dans l'intervalle allant de 4 à 50 % en poids, mieux encore de 4 à 20 % en poids, par rapport au poids total de la composition.

De préférence les compositions de l'invention contiennent au moins 4 % d'un ou plusieurs tensioactifs anioniques.

Les compositions selon la présente invention peuvent comprendre en outre au moins un tensioactif cationique.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,01 à 10 % en poids, mieux encore de 0,05 à 5 % en poids, et encore plus préférentiellement de 0,3 à 3 % en poids par rapport au poids total de la composition cosmétique.

De manière avantageuse, la composition selon l'invention comprend au moins un tensioactif anionique et au moins un tensioactif amphotère ou zwittérionique.

Les compositions selon la présente invention peuvent comprendre en outre au moins un polymère cationique.

Généralement la composition selon la présente demande comprend de 0,01 à 10 % en poids de polymère(s) cationique(s), de préférence de 0,1 à 5 % en poids, par rapport au poids total de la composition.

Les compositions selon l'invention peuvent comprendre, en outre, au moins un sel minéral ou organique tel que le chlorure de sodium.

Généralement la composition selon la présente invention comprend de 0,1 à 10 % en poids de sel(s), de préférence de 0,5 à 5 % en poids, par rapport au poids total de la composition.

Un mode de réalisation préféré consiste en ce que le ou les sels sont ajoutés à une composition selon l'invention comprenant au moins un ester d'acide gras en C₈ à C₁₄ et de sorbitan oxyéthyléné comprenant de 2 à 10 unités d'oxyde d'éthylène, au moins un monoester d'acide gras en C₈ à C₂₄ et de sorbitan oxyéthyléné comprenant de 15 à 50 unités d'oxyde d'éthylène, et au moins une cyclodextrine ou un de ses dérivés.

De manière préférée, l'ester d'acide gras en C₈ à C₁₄ et de sorbitan oxyéthyléné comprenant de 2 à 10 unités d'oxyde d'éthylène est un ester d'acide gras en C₁₂ et de sorbitan oxyéthyléné comprenant de 2 à 10 unités d'oxyde d'éthylène, et de préférence 4 unités d'oxyde d'éthylène. De manière particulièrement préférée, ledit ester est un mono-laurate de sorbitan oxyéthyléné à 4 OE.

De manière préférée, le monoester d'acide gras en C₈ à C₂₄ et de sorbitan oxyéthyléné comprenant de 15 à 50 unités d'oxyde d'éthylène est un monoester d'acide gras en C₈ à C₂₄ et de sorbitan oxyéthyléné à 20 OE. De manière particulièrement préférée, ledit monoester est un mono-laurate de sorbitan oxyéthyléné à 20 OE.

Les compositions selon l'invention présentent, de préférence, une viscosité dynamique allant de 2 à 8 Pa.s. et, de préférence, de 2,5 à 5,5 Pa.s., à température ambiante (25 °C). Ces mesures de viscosité peuvent notamment être effectuées à l'aide d'un rhéomètre de type Rhéomat R180, avec un intervalle de mesure de 30 s. et un taux de cisaillement de 200 mN.m.

Le milieu aqueux est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, l'hexylèneglycol, le propylèneglycol, les polyéthylèneglycols, et leurs mélanges.

Le pH des compositions selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

L'homme de métier veillera à choisir les éventuels additifs et leurs quantités de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

La composition selon l'invention peut être utilisée notamment comme composition de traitement ou de soin cosmétique des matières kératiniques.

Par matières kératiniques, on comprend les cheveux, les cils, les sourcils, la peau, les ongles, les muqueuses ou le cuir chevelu, et plus particulièrement les cheveux.

En particulier, la composition selon l'invention peut être utilisée comme shampooing ou composition à appliquer avant ou après un shampooing.

Un autre objet de l'invention est un procédé de traitement cosmétique, qui comprend l'application sur les cheveux d'une quantité efficace d'une composition cosmétique telle que décrite ci-dessus.

Selon un mode de mise en oeuvre préféré, un tel procédé consiste à appliquer sur les cheveux une quantité efficace de la composition cosmétique, à éventuellement rincer après un éventuel temps de pause.

Lorsque l'on applique la composition selon l'invention sous forme d'une lotion ou d'une crème avant ou après shampooing, on la laisse éventuellement pauser sur les cheveux pendant environ 1/2 minute à 5 minutes, puis on rince éventuellement à l'eau.

Les exemples suivants sont donnés à titre illustratif de la présente invention, et ne sauraient en limiter la portée.

### EXEMPLES

Dans les exemples suivants, les quantités sont indiquées en pourcentage pondéral de matière active (MA) par rapport au poids total de la composition, sauf indication contraire.

### EXEMPLE 1 :

On a réalisé la composition de shampooing A suivante :

| Composition | | A |
|---|---|---|
| Diméthylol-1,3-diméthyl-5,5-hydantoïne en solution aqueuse (à 55 % en poids) | | 0,14 |
| Sel de sodium du p-hydroxybenzoate de méthyle | | 0,2 |
| Chlorure d'hydroxypropylguar-triméthylammonium (Jaguar C 13 S commercialisé par Rhodia) | | 0,1 |
| Polymère carboxyvinylique (Carbopol 981 commercialisé par Noveon) | | 0,2 |
| Béta-cyclodextrine (cyclomatoheptaose) (Cavamax W 7 Pharma commercialisé par Wacker) | | 1,72 |
| Chlorure de sodium | | 0,1 |
| Polydiméthylsiloxane | | 2,7 |
| Mono-laurate de sorbitan oxyéthyléné à 4 OE (Tween 21 commercialisé par Uniqema) | | 6 |
| Cocoyl amidopropyl bétaïne en solution aqueuse (à 47 % en poids) | | 2,43 |
| Lauryl éther sulfate de sodium à 2,2 OE en solution aqueuse (à 70 % en poids) | | 15,5 |
| Parfum | | 0,5 |
| Hydroxyde de sodium pur/acide citrique, 1H₂O | q.s.p. | pH 6,5-7,5 |
| Eau désionisée | q.s.p. | 100 |

La composition A présente une excellente tolérance vis-à-vis du cuir chevelu. En particulier, on a observé très peu de réactions d'inconfort. En outre, cette composition présente une excellente tolérance oculaire.

Enfin, cette composition est stable et présente de bonnes qualités d'usage, ainsi que de remarquables propriétés cosmétiques.

### EXEMPLES 2 et 3 :

Les compositions de shampooing B et C suivantes ont été réalisées :

| Compositions | | B | C |
|---|---|---|---|
| Cocoyl amido bétaïne (Tégo Bétaïne F50 commercialisé par Goldschmidt Degussa) | | 2,4 | 2,4 |
| Diméthicone (Dow Coming 200 Fluid 500 000 cSt commercialisé par Dow Coming) | | 1,5 | 1,5 |
| Polyquaternium-10 (Ucare Polymer JR 400 LT commercialisé par Amerchol- Dow Chemical) | | 0,4 | 0,4 |
| Lauryl éther sulfate de sodium (Texapon AOS 225 UP commercialisé par Cognis) | | 15,4 | 15,4 |
| Béta-cyclodextrine (Cavamax W7 Pharma commercialisé par Wacker) | | 1,7 | 1,7 |
| Polysorbate 21 (Tween 21 commercialisé par Uniqema) | | 6 | 2 |
| Polysorbate 20 (Tween 20 commercialisé par Uniqema) | | - | 4 |
| Carbomer (Carbopol 980 commercialisé par Noveon) | | 0,2 | 0,2 |
| Chlorure de sodium ^{(*)} | | q.s. | q.s. |
| Conservateurs | | q.s. | q.s. |
| Acide citrique / Hydroxyde de sodium | q.s.p. | pH 5-5,6 | pH 5-5,6 |
| Eau désionisée | q.s.p. | 100 | 100 |

| | | | |
|---|---|---|---|
| ^{(*)} : ajouté en une quantité suffisante pour obtenir une viscosité allant de 3 à 5,5 Pa.s. à température ambiante (environ 25 °C), ladite viscosité étant mesurée à l'aide d'un rhéomètre Rhéomat R180, avec un intervalle de mesure de 30 s. et un taux de cisaillement de 200 mN.m. | | | |

Les compositions B et C présentent un aspect nacré, visible à l'oeil nu.

## Revendications

1. Composition cosmétique pour le soin des matières kératiniques comprenant, dans un milieu aqueux : au moins une cyclodextrine ou un de ses dérivés, et au moins un ester d'acide gras en C₈ à C₁₄ et de sorbitan oxyéthyléné comprenant de 2 à 10 unités d'oxyde d'éthylène.

2. Composition selon la revendication précédente, **caractérisée en ce** l'ester d'acide gras en C₈ à C₁₄ et de sorbitan oxyéthyléné est un ester d'acide gras en C₁₂ et de sorbitan oxyéthyléné comprenant de 2 à 10 unités d'oxyde d'éthylène.

3. Composition selon la revendication précédente, **caractérisée en ce** l'ester d'acide gras en C₈ à C₁₄ et de sorbitan oxyéthyléné est le mono-laurate de sorbitan oxyéthyléné à 4 OE.

4. Composition selon l'une quelconque des revendications précédentes, **caractérisée en ce qu'**elle comprend au moins 0,5 % en poids d'ester d'acide gras en C₈ à C₁₄ et de sorbitan oxyéthyléné comprenant de 2 à 10 unités d'oxyde d'éthylène par rapport au poids total de la composition.

5. Composition selon la revendication 4, **caractérisée en ce qu'**elle comprend de 0,5 à 10 % en poids d'ester d'acide gras en C₈ à C₁₄ et de sorbitan oxyéthyléné comprenant de 2 à 10 unités d'oxyde d'éthylène, plus préférentiellement de 2 à 9 % en poids, par rapport au poids total de la composition.

6. Composition selon l'une quelconque des revendications précédentes, **caractérisée en ce** la cyclodextrine est choisie parmi l'α-cyclodextrine, la β-cyclodextrine, la γ-cyclodextrine et l'un de leurs dérivés.

7. Composition selon la revendication précédente **caractérisée en ce que** la cyclodextrine est choisie parmi la β-cyclodextrine, la γ-cyclodextrine, de manière préférée il s'agit de la β-cyclodextrine.

8. Composition selon l'une quelconque des revendications précédentes, **caractérisée en ce** la cyclodextrine ou l'un de ses drivés représente de 1 à 15 % en poids, de préférence de 1 % à 10 % en poids, et encore plus préférentiellement de 1,5 % à 5 % par rapport au poids total de la composition.

9. Composition selon l'une quelconque des revendications précédentes, **caractérisée en ce qu'**il s'agit d'une composition lavante.

10. Composition selon la revendication précédente **caractérisée en ce qu'**elle comprend une teneur totale en tensioactifs d'au moins 4% en poids, de préférence entre 4% et 50% en poids par rapport au poids total de la composition.

11. Composition selon l'une quelconque des revendications précédentes, **caractérisée en ce qu'**elle comprend également au moins un monoester d'acide gras en C₈ à C₂₄ et de sorbitan oxyéthyléné comprenant de 15 à 50 unités d'oxyde d'éthylène.

12. Composition selon la revendication précédente, **caractérisée en ce que** ledit monoester est un monoester d'acide gras en C₈ à C₂₄ et de sorbitan oxyéthyléné comprenant 20 unités d'oxyde d'éthylène, de préférence le mono-laurate de sorbitan oxyéthyléné à 20 OE.

13. Composition selon l'une quelconque des revendications 11 ou 12, **caractérisée en ce qu'**elle comprend de 0,1 à 10 % en poids de monoester d'acide gras en C₈ à C₂₄ et de sorbitan oxyéthyléné comprenant de 15 à 50 unités d'oxyde d'éthylène, de préférence de 0,5 à 5 % en poids, par rapport au poids total de la composition.

14. Composition selon l'une quelconque des revendications précédentes, **caractérisée en ce qu'**elle comprend en outre au moins un tensioactif additionnel anionique et/ou non ionique et/ou amphotère ou zwittérionique.

15. Composition selon la revendication 14, **caractérisée en ce qu'**elle comprend au moins un tensioactif anionique et au moins un tensioactif amphotère ou zwittérionique.

16. Composition selon l'une quelconque des revendications 14 ou 15, **caractérisée en ce que** le tensioactif anionique est choisi parmi les alkylsulfates, les alkyléthersulfates et leurs mélanges.

17. Composition selon l'une quelconque des revendications 14 à 16, **caractérisée en ce que** la quantité du ou des tensioactifs anioniques est comprise dans l'intervalle allant de 0,5 à 50 % en poids, mieux encore de 4 à 20% en poids par rapport au poids total de la composition.

18. Composition selon l'une quelconque des revendications 14 ou 15, **caractérisée en ce que** le tensioactif amphotère ou zwittérionique est choisi parmi les (alkyl en C₈₋₂₀)-bétaïnes, les (alkyl en C₈-C₂₀)-amido(alkyl en C₆-C₈)bétaïnes et leurs mélanges.

19. Composition selon l'une quelconque des revendications précédentes, **caractérisée en ce qu'**elle comprend en outre au moins un tensioactif cationique.

20. Composition selon l'une quelconque des revendications précédentes, **caractérisée en ce qu'**elle comprend en outre au moins un polymère cationique.

21. Composition selon la revendication 20, **caractérisée en ce qu'**elle comprend de 0,01 à 10 % en poids de polymère(s) cationique(s), de préférence de 0,1 à 5 % en poids, par rapport au poids total de la composition.

22. Composition selon l'une quelconque des revendications précédentes, **caractérisée en ce que** le milieu aqueux est constitué d'eau ou d'un mélange d'eau et d'au moins un solvant cosmétiquement acceptable.

23. Composition selon la revendication 22, **caractérisée en ce que** le solvant est choisi parmi les alcools inférieurs en C₁-C₄ et les polyols.

24. Composition selon l'une quelconque des revendications précédentes, **caractérisée en ce qu'**elle comprend en outre un ou plusieurs additifs classiques, tels que les agents antichute, les agents oxydants, les céramides et pseudo-céramides, les vitamines et pro-vitamines dont le panthénol, les huiles végétales, animales, minérales ou synthétiques, les cires, les céramides et pseudo-céramides, les filtres solaires, les pigments minéraux ou organiques, colorés ou non colorés, les colorants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

25. Utilisation de la composition selon l'une quelconque des revendications 1 à 24, comme composition de traitement ou de soin cosmétique des matières kératiniques.

26. Utilisation de la composition selon l'une quelconque des revendications 1 à 24, comme shampooing ou composition à appliquer avant ou après un shampooing.

27. Procédé de traitement cosmétique, comprenant l'application sur les cheveux d'une quantité efficace d'une composition cosmétique selon l'une quelconque des revendications 1 à 24.
